# EUROPEAN PATENT SPECIFICATION

(11) **EP 2 571 555 B1**
(45) Date of publication and mention of the grant of the patent: **05.11.2014**
(21) Application number: 11719837.4
(22) Date of filing: 17.05.2011
(51) Int. Cl.: A61M 16/20, H01L 41/053, F03G 7/06

(54) **MECHANICAL TEMPERATURE COMPENSATION MEANS, METHOD FOR ASSEMBLY SAID MEANS AND METHOD FOR MECHANICALLY TEMPERATURE COMPENSATING**
MITTEL ZUM MECHANISCHEN TEMPERATURAUSGLEICH, VERFAHREN ZUR MONTAGE BESAGTER MITTEL UND VERFAHREN ZUM MECHANISCHEN TEMPERATURAUSGLEICH
MOYENS DE COMPENSATION DE TEMPÉRATURE MÉCANIQUES, PROCÉDÉ POUR ASSEMBLER LESDITS MOYENS ET PROCÉDÉ POUR COMPENSER MÉCANIQUEMENT UNE TEMPÉRATURE

(30) Priority: 18.05.2010 US 345756 P; 18.05.2010 US 345797 P; 17.05.2010 SE 1050488; 17.05.2010 SE 1050478
(43) Date of publication of application: 27.03.2013
(73) Proprietor: Mindray Medical Sweden AB, 174 57 Sundbyberg (SE)
(72) Inventor: CEWERS, Göran, S-216 42 Limhamn (SE)
(74) Representative: KIPA AB
(86) International application number: PCT/EP2011/057965
(87) International publication number: WO 2011/144610

(56) References cited:
- WO-A1-2005/026529
- DE-A1- 10 303 855
- US-A1- 2009 278 631

## Description

### Related applications

The present application relates to the following applications of the same inventor as the present application with the following titles: "VALVE AND METHOD TO CONTROL A FLOW THROUGH THE VALVE" (US61/345,623); "MECHANICAL AMPLIFIER, SYSTEM OF SAID AMPLIFIERS AND METHOD FOR MECHANICALLY AMPLIFICATION OF A MOTION" (US61/345,625); "VALVE AND METHOD TO CONTROL A FLOW" (US61/345,628); "MECHANICAL TRIMMING DEVICE AND TRIMMING METHOD" (US61/345,733); "MECHANICAL TEMPERATURE COMPENSATION MEANS, METHOD FOR ASSEMBLY SAID MEANS AND METHOD FOR MECHANICALLY TEMPERATURE COMPENSATING" (US61/345,756).

### Background of the invention

### Field of the Invention

The disclosure pertains to a device and a method for compensating thermal expansion in solid materials such as plastic, metal or ceramics. The disclosure also comprises a method for operating and manufacturing such a device. The deivce may for instance be applied in piezoceramic driven valves of medical ventilators.

### Description of Related Art

When designing mechanical systems it is common for components of different materials to be used in the construction. The reasons may be special requirements for the parts used such as hardness, fatigue properties, corrosion resistance, surface roughness, transparency, colour, electrical properties, melting point, cost etc.

When different materials are combined it is common for the parts being used to have different coefficients of thermal expansion. If the structure can be constructed to facilitates if thermal expansions should occur, without damaging the structure or functionality, this does not cause any problems. For example, these issues may be avoided by having adequate tolerances, allowing for the thermal effects. This combined with a construction having a structure and a material choice having as little impact as possible when temperature may impact the functionality.

However, there are temperature critical structures where temperature compensation elements are required for a satisfactory operation when the temperatures are fluctuating.

Example of where temperature critical structures may be included are devices for: micro-positioning, controlling laser beams, microscope focusing - atomic, optical and ultrasound - manufacturing of semiconductors, sensors for micro-positioning, spectroscopy and optical benches.

In micro-positioning it is common for the positioning to be controlled by an actuator, such as a piezoelectric crystal. A piezoelectric crystal has an actuation range of approximately 0.1% of the crystal thickness, i.e. the actuation range is extremely small in relation to the crystal thickness. To obtain a greater motion, structures have been produced in the form of bimorphous crystals which have two layers of piezo materials with opposite working directions. Bending is obtained in the same way as for bimetals by constructing joined layers into beams. On the other hand, the piezo beam is heat stable as it comprises materials with the same thermal expansion coefficients throughout.

However, the disadvantage of piezo beams is that the force is limited by the fragility of the piezo ceramics.

Another method for obtaining greater motion using piezo technology is to connect a number of piezo elements in series forming a stack.

These may be made integrated which may be done in a similar manner to the manufacturing of ceramic multilayer condensers. A piezoactuator having a length of several centimetres may be made in this way. If a stack is fastened at one end, the other end will move relative to its surroundings of the other end. The surroundings of the other end usually comprising the same material which is fastened in the end of the piezo stack. In order to obtain a relative motion not being influenced by the ambient temperature, the piezo stack and the surrounding material must have the same thermal expansion coefficient. However, this is hard to achieve, as the piezo material often has thermal expansion coefficients of just a few ppm, and even a negative coefficient in some materials. The surrounding material must have the same coefficient, which highly restricts the possible choice of materials. Only certain special alloys and ceramic materials remain as possible options. These options are often unsuitable due to strength, manufacturing methods, corrosion properties or high cost.

The solutions available today are e.g. for a second piezo stack to act as reference point to the first. This adds to the cost and places restrictions on mechanical design. Another method is to use special materials to a reference point. Yet another method is to place an element with significant heat expansion in series with the piezo stack. The piezo stack in series with the compensation element could then be made to have the same heat expansion as the surrounding materials. This principle is described in US patents 7,514,847 and 6,148,842.

US patent 7,514,847 uses an aluminium body as compensation component. As aluminium has relatively low thermal expansion coefficient compared with ordinary construction materials, a larger body of this kind is needed, resulting in significantly increased dimensions and an impaired response times.

US patent 6,148,842 uses a closed container filled with oil as compensation body. This solution provides a compact compensation body as there are oils with high thermal expansion coefficients. The disadvantage is that the oil must be enclosed hermetically in order to avoid leakage, resulting in high manufacturing costs.

Compensation methods using memory metal are described, e.g. in US patent 5,059,850.

However, this solution is beset by hysteretic problems, material choices and high costs.

Further examples are found in DE 10303855 and WO 2005/026529.

One object of the invention is thus to provide a device and/or method for mechanical temperature compensation in a device composing structures and materials with different thermal expansion coefficients. In particular, such device should advantageously have a simple design with low manufacturing costs and small dimensions.

### Summary of the Invention

These objects are met by means of the device and the method according to the appended independent claims, while particular embodiments of are are subject of the dependent claims.

Accordingly, embodiments of the present invention seek primarily to mitigate, alleviate or eliminate one or more of the above-identified deficiencies or disadvantages in the art, singly or in any combination, and solve at least partly the abovementioned issues by providing a device and method according to the appended patent claims.

In a first aspect, the invention may be described as a mechanical temperature compensation element intended to be used as a compensation element for heat expansion. The element comprises a flat element with a first thermal expansion coefficient, a housing with a second thermal expansion coefficient different from the first thermal expansion coefficient, a, in relation to the flat element, inclined linkage device which mechanically connecting the flat element and the housing; when the temperature changing the flat element expands radially and the linkage device is moved radially, wherein the radial expansion from the flat element is converted to an, relative to the flat element, orthogonal movement, which raises or lowers the housing depending on the temperature of the temperature compensation element.

This design provides a mechanical device which can be used to mechanically compensate for changes depending on temperature changes and which may be used for temperature critical structures such as for micro-positioning, controlling of laser beams, microscope focusing - atomic, optical and ultrasound - semiconductor manufacturing, sensors for micro-positioning, spectroscopy and optical benches. Or to compensate for the temperature dependent, stroke length of a piezo element.

The temperature compensating is obtained by the flat material having a thermal coefficient being higher or lower than an upper laying housing or a housing made of two opposed halves. When a temperature change occurs, the flat element is expanded radially, which results in, a to the housing connected, mechanical device executing a lever-like movement and raising and lowering the housing orthogonally relative to the flat element.

The flat element and the housing may have varying shapes in various embodiments. For example, they can either be circular shaped, polygonal shaped or ellipsoid shaped.

In one embodiment of the mechanical temperature compensation element, the link device comprising a disk element, such as a washer, with a rhomboidal cross-section, radial slits and/or separate segments with rhomboidal cross-section.

It is through this design, of the mechanically linked device between the flat element and the housing that results in the lever-like movement which is caused by the flat element's temperature dependent radial change.

In yet another embodiment of the mechanical temperature compensation element, the flat element has a thermal expansion coefficient higher than that of the housing.

This provides positive temperature compensation, which provides a raising effect when the temperature increases. Examples of a material that my be used for the flat element is Zinc.

In yet another embodiment of the mechanical temperature compensation element, the flat element has a thermal expansion coefficient lower than that of the housing.

This provides negative temperature compensation, which causes the mechanical temperature compensation element to lowers when the temperature increases.

In yet another embodiment of the mechanical temperature compensation element, it may be connected in series to a piezo element.

In a connection of this kind, the mechanical temperature compensation element is used to compensate for temperature dependent changes in the stroke length of the piezo element. But as already mentioned, the invention may be used to temperature compensate in other temperature critical structures.

Another aspect of the invention comprising a method for assembling the mechanical temperature compensation element comprising cooling down the flat element prior to assembly.

A third aspect of the invention comprising a method for assembling the mechanical temperature compensation element, comprising heating the housing prior to assembly.

These two aspects of the invention comprising heating or cooling components so that a pressure fit occurs when the components are mounted and that the temperature of the components are controlled to the intended temperature compensation range.

A fourth aspect of the invention comprising a method for mechanically temperature compensating a temperature critical structure, comprising a flat element having a thermal expansion coefficient which is different from the thermal expansion coefficient of its housing, at temperature changes is expanded and thus presses on a, between the flat element and the housing, mechanically linked device raising or lowering the housing orthogonally relative to the flat element interacting with the temperature critical structure.

Yet another aspect of the invention provides a temperature compensation method wherein a temperature compensation element is used as an element for compensating for heat expansion whereby a flat element is expanded radially upon changes in temperature and an inclined linkage device is moved radially whereupon the radial expansion of the flat element is converted to an orthogonal movement in relation to the flat element raising or lowering a housing surrounding the flat element depending on the temperature of the temperature compensation element.

Yet a further aspect is the use of the presented temperature compensation element or the temperature compensation method, for compensating a temperature critical assembly.

The advantages of this method are the same as for the equipment described above - having a relatively simple and inexpensive way of obtaining mechanical temperature compensation in a temperature critical assembly such as a piezo element.

Further embodiments of the invention are defined in the dependent claims, wherein features for the second and subsequent aspects of the invention are as for the first aspect mutatis mutandis.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which the invention at least is capable of will be apparent and elucidated from the following description of embodiments of the present invention, reference being made to the accompanying drawings, in which
Figure 1 is a schematic view showing an exemplary embodiment according to a principle of the invention. In this example the device has positive temperature compensation and is at its lowest working temperature;
Figure 2 is the same exemplary embodiment as in Figure 1, but with the device at its highest working temperature;
Figure 3 is showing an embodiment where one can obtain negative temperature compensation in accordance with the invention - in this example the device is at its highest working temperature;
Figure 4 is a detailed view of Figure 2 showing the principle of the link device;
Figures 5 and 6 are showing variants of the link device; and
Figures 7 and 8 are flow-charts illustrating the method of temperature compensation and a method for manufacturing the temperature compensation means.

### Description of Embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

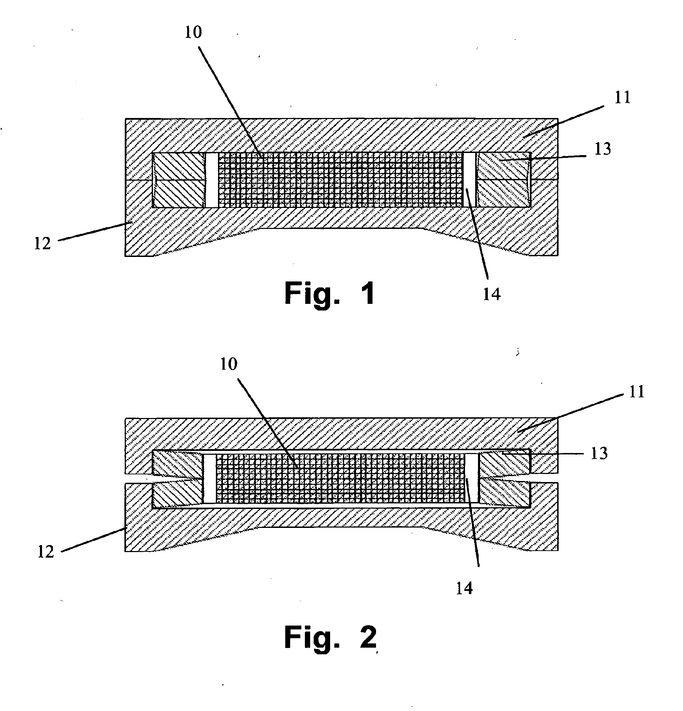

Figure 1 illustrates a device according to an embodiment of the invention. The device may be provided by a disc 10 having a relatively high thermal expansion coefficient. The disc 10 is enclosed by a housing 11,12. The housing 11, 12 has two parts enclosing the disc 10.

In some embodiments, the housing 11, 12 comprises two discs, each having a cavity. Alternatively, only one of the housing parts 11, 12 has a cavity.

The discs are mounted so that the cavities receive disc 10 inside the housing. Disc 10 is in tension with a link device 13, which may be a washer with rhomboidal cross-section, as shown in Fig. 1. The disc 10 has a high thermal expansion coefficient compared to the housing 11, 12.

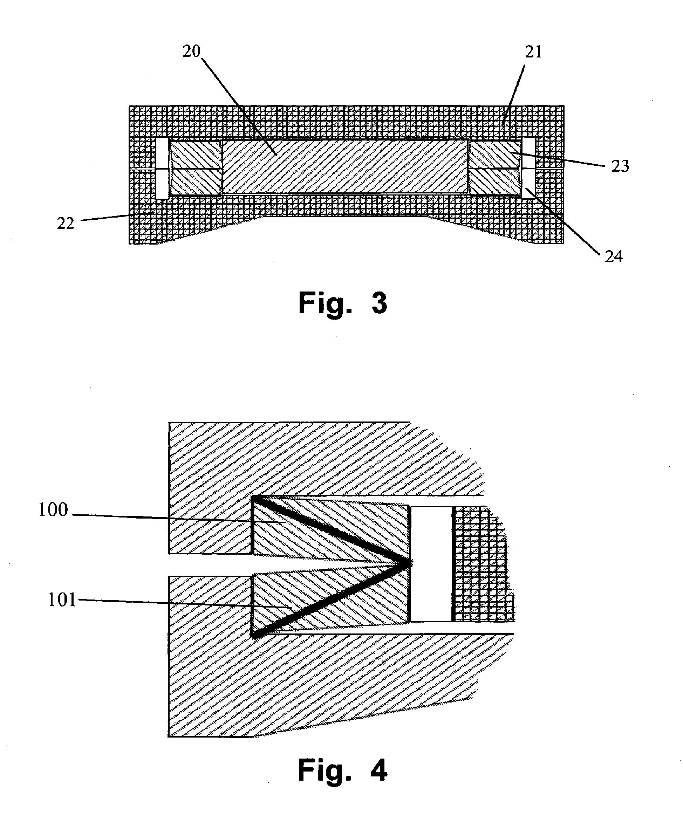

When the temperature increases disc 10 expands more radially than along the axis, since the diameter of the disc is greater than the thickness. Moreover, disc 10 expands more than the housing 11, 12, whereupon the link device 13 exposes housing 11, 12 to a radial pressure. In the exemplary embodiment, link device 13 comprising two rings with rhomboidal cross-section and exerts a radial pressure on housing 11, 12. The rhomboidal cross-section of link device 13 is given a function of an inclined supporting element (e.g. a strut) 100, 101, as illustrated in Fig. 4, with the diagonal lines in the cross-section of the link device 13. The radial movement caused by the heat expansion of disc 10 is converted in an axial movement having an amplification factor determined by the inclination of lines 100, 101, i.e. the design of the link device 13 according to desired specifications and applications.

In Fig. 1 the device at the lowest working temperature is showed and in Fig. 2 at the highest working temperature. At typical working temperature parts 11, 12 of the housing are separated by a distance of preferably half of what is illustrated in figure 2. At the same time, the link device 13 is only in contact with the disc 10 and housing 11, 12 at the corner of the opposing diagonals 100,101, shown in Fig. 4.

Additionally and or alternatively, if a ring 14 is positioned between the disc 10 and the link device 13, as in figure 1 and 2. The link device 13 will only be in contact with ring 14 and the housing 11, 12 at the corner of the opposing diagonals 100,101, as shown in Fig. 4.In this position, as well as the highest working temperature, the whole device is being held together as a continuous unit by pressure fit caused by radial pressure against the link device 13 from the disc 10.

In order for the device to withstand high axial counter forces, the radial surface of the disc 10 may be surrounded by a thin ring 14. The ring 14 is preferably made of a hard material. This prevents the shape of the disc 10 from being deformed, even if it consists of a material being softer than the housing. By choosing zinc as material for the disc 10 and stainless steel for the rest of the device, for example, a thermal expansion coefficient of 150 ppm/degree may be obtained. This is approximately 10 times higher than most structural materials. By varying the angle of the rhomboidal shape of link device 13, the mechanical amplification and thus the axial heat expansion coefficient may be determined as desired.

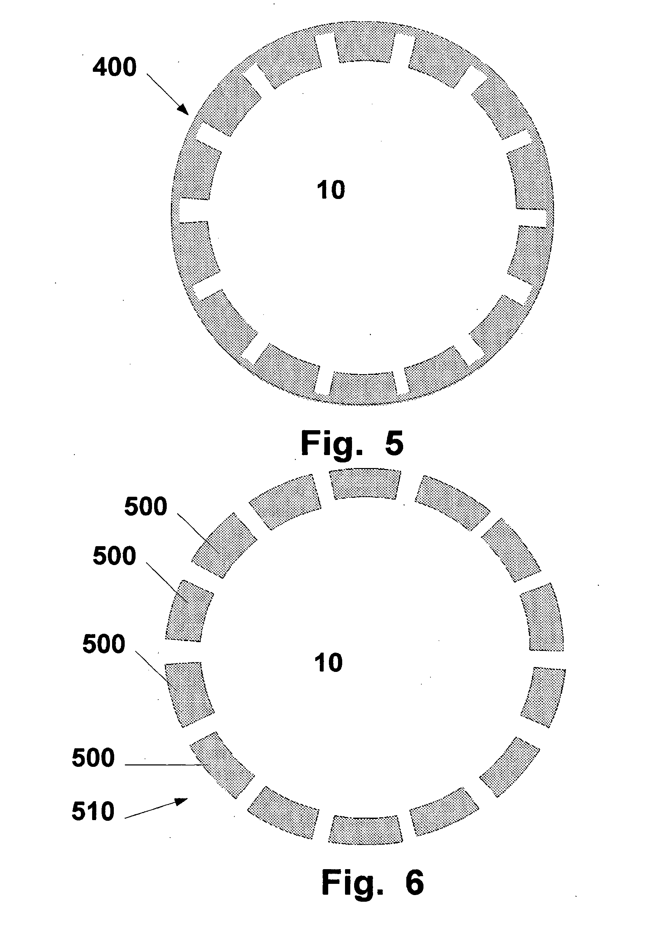

When the link device 13 is exposed to radial forces it will exert a counter force on the disc 10. This will compress the disc by, in practice, a reduced heat expansion effect. In order to reduce this effect, link device 13 may be equipped with slits as shown in Fig. 5, or may comprise of loose segments as shown in Fig. 6. Slits in link device 13 may comprise of none penetrating radial grooves.

By using a material having a high thermal expansion coefficient in the housing 11,12 and in the rest of the device using material having a low thermal expansion coefficient relative to it, as shown in Fig. 3, a negative thermal expansion coefficient may be obtained. In Fig.3 21, 22 refers to the housing, 20 refers to the expandable disc and 23 to the linkage device.

Additionally in some embodiments of the device illustrated in Fig. 3, a ring 24, forming a hard surface against housing 21, 22, may be placed in a slightly different position compared ring 14 in Fig. 2.

In some embodiments, the parts used are circular, but the device is not restricted to these shapes. The circular geometry of the device, t may be changed to polygons, ellipses or similar shapes.

Fig. 5 and 6 are illustrating different variants of the linkage device. As shown in Fig. 5, the linkage device may be arranged in a continuous ring 400. Alternatively, as shown in Fig. 6 individual elements 500 may be arranged adjacent each other, radially equidistant offset, in a circle 510 around the disc 10.

Additionally, in some embodiments the temperature compensation means may be connected in series to a trimming device.

Details on suitable mechanical trimming device and trimming method for adjustment of dimensional tolerances can be found in US patent applications numbers US61/345,733 and US 13/106,461 of the same inventor, which is incorporated herein by reference in its entirety for all purposes.

The mechanical trimming device and methods are well suitable for the present embodiments of mechanically temperature compensation means, in accordance with the following reasoning. The mechanical trimming device is performed by mechanically working together with the device for which the dimension tolerances are to be compensated.

The mechanical trimming is performed by screwing a screw through a threaded hole. The ends of the screw pressing against a centre of a disk member or washer, which by deformation acts as a lever against an adjacent body. This reduces the effect of the lead of the screw to the transmission determined by the abovementioned lever and the adjacent body is moved a substantially shorter distance than the screw.

In one aspect of the US61/345,733 disclosure includes a mechanical trimming device comprising an adjustable; a first body having at least one threaded through hole that rotatable partly enclosing said adjustable screw; a movable second body, that is movable relative to the first body, and has a cavity into the second body. The cavity accommodates a proximal end of said screw. When the second body is laid against said first body; at least one disc member having a centre and an outer-edge and is positioned between the first and the second bodies. The cavity is facing the disc member and has an edge lying inside of the outer-edge of the disc member so that the outer edge of the disc member lies between the first and the second body.

The screw, when is adjusted, can raise the centre of the disc member in that the disc member works as a lever having a first distributed contact point against the cavity edge of the second body and a second distributed contact point along the outer-edge of the disc member against the surface of the first body, wherein the first body is moved in the axial direction of the screw with a substantially smaller motion than the screw.

This device provides the possibility of using a screw to adjust the second, relative to the first body movable, body, e.g. orthogonally relative to the surface of the first body. The orthogonal adjustment of the second body, occurring when the screwing the screw, is due to the design of the device extremely small in relation to the motion of the screw. Preferably the adjustment is in the micrometre range. The adjustment using the abovementioned device may be performed either vertically or horizontally depending on the positioning of the device. When trimming, the second body is moved along the axial direction of the screw with a movement substantially less than the screw.

When the trimming has been completed the disc member locks the adjustment screw in its position provided there is a counter force through the second body keeping the disc member in a pre-stressed position. Thus the trimming cannot come undone.

This provides a cost effective and simple way to obtain a device usable for trimming by micro-positioning, such as controlling laser beams, microscope focusing; atomic, optical and ultrasound, semiconductor manufacturing, sensors for micro-positioning, spectroscopy and optical benches.

For example, the device may be used for mechanical trimming of the stroke length of a piezoelectric crystal stack, but also for other types of actuator unit.

Additionally and/or alternatively, in some embodiments, the mechanical trimming device has disc member slits running towards the centre. In some embodiments may the disc member also including towards the centre running sectioned elements. The disc member may be circular in shape but may also be polygonal. However, the geometry is not restrained to these shapes, but may also be ellipsoids or similar.

The disc member may be designed in many ways as long as the general principles described here within are complied with. For example, the disc member may have a hole in the centre where the slits meet. It may also be large sections. The disc member may also be designed with a solid centre, where the screw presses, with outwards directing sections or arms.

Additionally and/or alternatively, in some embodiments of the mechanical trimming device, the mechanical trimming device is connected in series to a mechanical temperature compensation element.

This configuration of the device also provides, if required, compensation for changes in the ambient temperature, e.g. the stroke length of an actuator unit, preferably a piezoactuator.

Additionally and/or alternatively, in some embodiments, the mechanical trimming device may be connected in series to an actuator unit and to a mechanical temperature compensation element, if necessary.

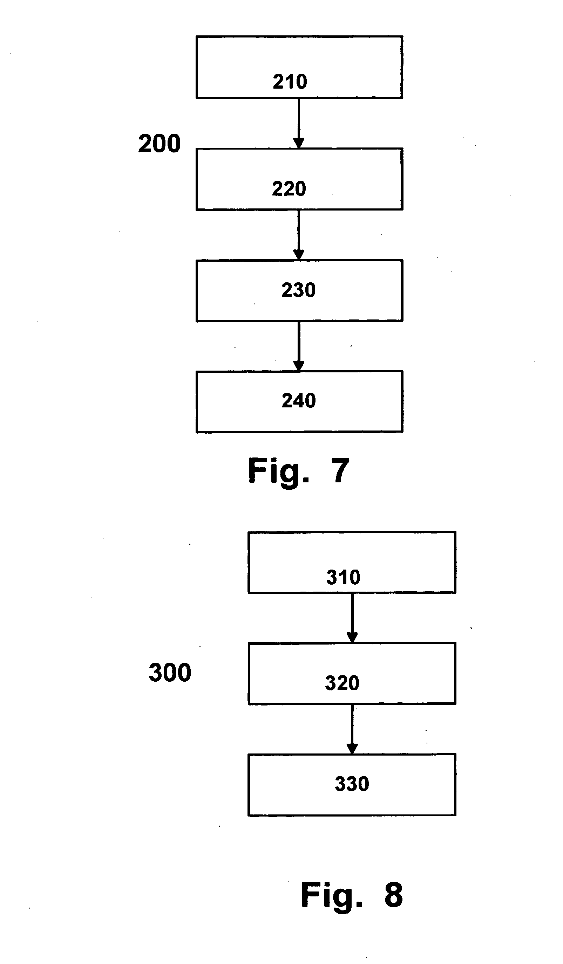

In Fig. 7 is a flow-chart shown illustrating a manufacturing method 200 for one embodiment of the above described device.

As already mentioned, the entire device is held together by a pressure fit. By applying temperatures lower than the working temperature range of the disc 10, all components can be mounted without difficulty.

An advantageous method of assembly is to cool down disc 10, 20 to a low temperature 210 prior to the assembly, e.g. in liquid nitrogen. After assembly the device is exerted to axial pressure 220 and is allowed to be temperature equalised 230 until the device reaches its working temperature range, after which the pressure may be removed 240.

When assembling a device having negative heat expansion coefficient, as shown in Fig. 3, the housing 21, 22 is heated up instead, thereafter is the device exerted to axial pressure and the temperature is allowed to be equalised until the device reaches its working temperature range, where after the pressure can be removed.

In Fig. 8 is a flow-chart shown, illustrating a method for temperature compensation 300 wherein a temperature compensation element is used as an compensation element for heat expansion. The method comprising:
expanding a flat element 310 radially upon changes in temperature; moving an inclined linkage device radially (320); wherein the radial expansion of the flat element is converted to an orthogonal movement 330 in relation to the flat element raising or lowering a housing enclosing the flat element depending on the temperature of the temperature compensation element.

Additionally and/or alternatively, connecting the temperature compensation element in series to a piezo element.

Additionally and/or alternatively, positioning a ring element between the flat element and the linkage device.

Additionally and/or alternatively, positioning a ring element between the linkage device and the housing.

The ring element is used to avoid deformation of a material being softer than the linkage device.

A mechanical temperature compensation element as described above, may advantageously be used in medical device applications due to high requirements regarding reliability, energy efficiency, and patient safety.

A mechanical temperature compensation element as described above, may advantageously be used in a valve of a medical ventilator, such as described in US61/345,797, which is incorporated herein by reference in its entirety.

The present invention has been described above with reference to specific embodiments. However, other embodiments than the above described are equally possible within the scope of the invention.

The scope of the invention is only limited by the appended patent claims.

## Claims

1. A mechanical temperature compensation element, in particular intended to be used as a compensation element for heat expansion, comprising:
- a flat element (10, 20) having a first thermal expansion coefficient;
- a housing (11, 12, 21, 22) having a second thermal expansion coefficient different from said first thermal expansion coefficient;
- a, in relation to said flat element, inclined linkage device (13, 23) which mechanically connects said flat element and said housing;
- wherein at temperature changes said flat element (10, 20) expands radially and said linkage device is moved radially, wherein said radial expansion from said flat element is converted to an, relative to said flat element, orthogonal movement, which raises or lowers said housing depending on the temperature of the temperature compensation element.

2. The temperature compensation element according to claim 1, wherein said housing is two opposed halves.

3. The temperature compensation element according to claim 1 or 2, wherein said flat element is a circular shaped disc or wherein said flat element is a polygon.

4. The temperature compensation element according to any of claims 1-3, wherein said housing is circular or wherein said housing is polygon shaped.

5. The temperature compensation element according to any of claims 1-4, wherein said linkage device comprising a washer having a rhomboidal cross-section; and/or
wherein said linkage device comprises radial slits; and/or wherein said linage device comprising, separate segments having rhomboidal cross-section.

6. The temperature compensation element according to any of claims 1-5, wherein said thermal extension coefficient of said flat element is higher than that of said housing; or
wherein said thermal extension coefficient of said flat element is lower than that of said housing.

7. The temperature compensation element according to any of claims 1-6, wherein said flat element is made of zinc.

8. The temperature compensation element according to any of claims 1-7, wherein said temperature compensation element is connected in series to a piezo element.

9. The temperature compensation element according to claims 1-8, wherein a ring element (14, 24) is positioned between said flat element (10, 20) and said linkage device (13, 23); and/or between said linkage device (13, 23) and said housing (11, 12, 21, 22).

10. The temperature compensation element according to claim 9, wherein said ring element (14, 24) is of a rigid, hard material to prevent said disc (10, 20) from being deformed.

11. A method for manufacturing of a temperature compensation element (200) according to claim 1-10 comprising: cooling down or heating up said flat element (10, 20) before mounting (210), as well as exerting the device to axial pressure after mounting (220)and to temperature equalize until said device reaches its temperature working range (230), whereupon said pressure is removed (240).

12. The method according to claim 11, for assembling a temperature compensation element according to claim 6, comprising cooling down said flat element prior to being mounted.

13. The method according to claim 11, for assembling a temperature compensation element according to claim 6, comprising heating up said housing prior to being mounted.

14. A method for temperature compensation (300)
wherein a temperature compensation element is used as an compensation element for heat expansion comprising:
expanding a flat element (310) radially upon changes in temperature;
moving an inclined linkage device radially (320);
wherein the radial expansion of said flat element is converted to an orthogonal movement (330) in relation to said flat element raising or lowering a housing enclosing said flat element depending on the temperature of said temperature compensation element.

15. Use of a temperature compensation element according to any of claim 1 to 10 or the temperature compensation method according to claim 14, for compensating a temperature critical assembly.

## Patentansprüche

1. Mechanisches Temperaturausgleichselement, das insbesondere zur Verwendung als ein Ausgleichselement für Wärmeausdehnung gedacht ist, das umfasst:
- ein ebenes Element (10, 20) mit einem ersten Wärmeausdehnungskoeffizienten;
- ein Gehäuse (11, 12, 21, 22) mit einem zweiten Wärmeausdehnungskoeffizienten, der sich von dem ersten Wärmeausdehnungskoeffizienten unterscheidet;
- eine in Bezug auf das ebene Element geneigte Verbindungsvorrichtung (13, 23), die das ebene Element und das Gehäuse mechanisch verbindet;
- wobei sich bei Temperaturänderungen das ebene Element (10, 20) radial ausdehnt, und die Verbindungsvorrichtung radial bewegt wird, wobei die radiale Ausdehnung des ebenen Elements in eine in Bezug auf das ebene Element orthogonale Bewegung umgewandelt wird, die das Gehäuse in Abhängigkeit von der Temperatur des Temperaturausgleichselements anhebt oder absenkt.

2. Temperaturausgleichselement nach Anspruch 1, wobei es sich bei dem Gehäuse um zwei einander gegenüberliegende Hälften handelt.

3. Temperaturausgleichselement nach Anspruch 1 oder 2, wobei das ebene Element eine kreisförmige Platte ist, oder wobei das ebene Element ein Polygon ist.

4. Temperaturausgleichselement nach einem der Ansprüche 1 bis 3, wobei das Gehäuse kreisförmig ist, oder wobei das Gehäuse die Form eines Polygons aufweist.

5. Temperaturausgleichselement nach einem der Ansprüche 1 bis 4, wobei die Verbindungsvorrichtung eine Unterlegscheibe mit einem rautenförmigen Querschnitt aufweist, und/oder
wobei die Verbindungsvorrichtung radiale Schlitze aufweist, und/oder wobei die Verbindungsvorrichtung separate Segmente mit einem rautenförmigen Querschnitt aufweist.

6. Temperaturausgleichselement nach einem der Ansprüche 1 bis 5, wobei der Wärmeausdehnungskoeffizient des ebenen Elements höher als der des Gehäuses ist oder wobei der Wärmeausdehnungskoeffizient des ebenen Elements niedriger als der des Gehäuses ist

7. Temperaturausgleichselement nach einem der Ansprüche 1 bis 6, wobei das Gehäuse kreisförmig ist, oder wobei das ebene Element aus Zink besteht.

8. Temperaturausgleichselement nach einem der Ansprüche 1 bis 7, wobei das Temperaturausgleichselement in Reihe mit einem Piezoelement verbunden ist.

9. Temperaturausgleichselement nach einem der Ansprüche 1 bis 8, wobei ein Ringelement (14, 24) zwischen dem ebenen Element (10, 20) und der Verbindungsvorrichtung (13, 23) und/oder zwischen der Verbindungsvorrichtung (13, 23) und dem Gehäuse(11, 12, 21, 22) positioniert ist.

10. Temperaturausgleichselement nach Anspruch 9, wobei das Ringelement (14, 24) aus einem starren, harten Material besteht, um zu verhindern, dass sich die Platte (10, 20) verformt.

11. Verfahren zum Herstellen eines Temperaturausgleichselements (200) nach Anspruch 1 bis 10, das umfasst: Abkühlen oder Erwärmen des ebenen Elements (10, 20) vor der Montage (210) wie auch Ausüben eines axialen Drucks auf die Vorrichtung nach der Montage (220)und Ausgleichen der Temperatur, bis die Vorrichtung ihren Temperatur-Betriebsbereich (230) erreicht, woraufhin der Druck entfernt (240) wird.

12. Verfahren nach Anspruch 11 zum Zusammenbauen eines Temperaturausgleichselements nach Anspruch 6, das ein Abkühlen des ebenen Elements umfasst, bevor es montiert wird.

13. Verfahren nach Anspruch 11 zum Zusammenbauen eines Temperaturausgleichselements nach Anspruch 6, das ein Erwärmen des Gehäuses umfasst, bevor es montiert wird.

14. Verfahren zum Temperaturausgleich 300, wobei ein Temperaturausgleichselement als ein Ausgleichselement für Wärmeausdehnung verwendet wird, wobei das Verfahren umfasst:
radiales Ausdehnen eines ebenen Elements (310) bei Temperaturänderungen;
radiales Bewegen einer geneigten Verbindungsvorrichtung (320), wobei die radiale Ausdehnung des ebenen Elements in eine orthogonale Bewegung (330) in Bezug auf das ebene Element umgewandelt wird, durch die ein Gehäuse, welches das ebene Element umschließt, in Abhängigkeit von der Temperatur des Temperaturausgleichselements angehoben oder abgesenkt wird.

15. Verwenden eines Temperaturausgleichselements nach einem der Ansprüche 1 bis 10 oder des Temperaturausgleichsverfahrens nach Anspruch 14 zum Ausgleichen einer temperaturkritischen Baugruppe.

## Revendications

1. Elément de compensation de la température mécanique, en particulier destiné à être utilisé comme élément de compensation de la dilatation thermique, comprenant :
- un élément plat (10, 20) ayant un premier coefficient de dilatation thermique,
- un boîtier (11, 12, 21, 22) ayant un deuxième coefficient de dilatation thermique différent dudit premier coefficient de dilatation thermique,
- en relation avec ledit élément plat, un dispositif de liaison incliné (13, 23) qui connecte mécaniquement ledit élément plat et ledit boîtier,
- dans lequel lors de changements de température ledit élément plat (10, 20) se dilate radialement et ledit dispositif de liaison est déplacé radialement, dans lequel ladite dilatation radiale dudit élément plat est convertie, par rapport audit élément plat, en mouvement orthogonal, qui soulève ou abaisse ledit boîtier en fonction de la température de l'élément de compensation de la température.

2. Elément de compensation de la température selon la revendication 1, dans lequel ledit boîtier est constitué de deux moitiés opposées.

3. Elément de compensation de la température selon la revendication 1 ou 2, dans lequel ledit élément plat est un disque de forme circulaire ou dans lequel ledit élément plat est un polygone.

4. Elément de compensation de la température selon l'une quelconque des revendications 1 à 3, dans lequel ledit boîtier est circulaire ou dans lequel ledit boîtier est en forme de polygone.

5. Elément de compensation de la température selon l'une quelconque des revendications 1 à 4, dans lequel ledit dispositif de liaison comprend une rondelle ayant une section transversale rhomboïdale, et/ou
dans lequel ledit dispositif de liaison comprend des fentes radiales, et/ou
dans lequel ledit dispositif de liaison comprend des segments séparés ayant une section transversale rhomboïdale.

6. Elément de compensation de la température selon l'une quelconque des revendications 1 à 5, dans lequel ledit coefficient de dilatation thermique dudit élément plat est supérieur à celui dudit boîtier, ou
dans lequel ledit coefficient de dilatation thermique dudit élément plat est inférieur à celui dudit boîtier.

7. Elément de compensation de la température selon l'une quelconque des revendications 1 à 6, dans lequel ledit élément plat est fait de zinc.

8. Elément de compensation de la température selon l'une quelconque des revendications 1 à 7, dans lequel ledit élément de compensation de la température est relié en série avec un élément piézoélectrique.

9. Elément de compensation de la température selon l'une quelconque des revendications 1 à 8, dans lequel un élément en anneau (14, 24) est positionné entre ledit élément plat (10, 20) et ledit dispositif de liaison (13, 23), et/ou entre ledit dispositif de liaison (13, 23) et ledit boîtier (11, 12, 21, 22).

10. Elément de compensation de la température selon la revendication 9, dans lequel ledit élément en anneau (14, 24) est fait d'un matériau dur rigide pour empêcher ledit disque (10, 20) d'être déformé.

11. Procédé de fabrication d'un élément de compensation de la température (200) selon les revendications 1 à 10 comprenant : le refroidissement ou le chauffage dudit élément plat (10, 20) avant montage (210), ainsi que le fait d'exercer sur le dispositif une pression axiale après montage (220) et le fait d'égaliser la température jusqu'à ce que ledit dispositif atteigne sa plage de température de travail (230), après quoi ladite pression est supprimée (240).

12. Procédé selon la revendication 11, destiné à assembler un élément de compensation de la température selon la revendication 6, comprenant le refroidissement dudit élément plat avant qu'il soit monté.

13. Procédé selon la revendication 11, destiné à assembler un élément de compensation de la température selon la revendication 6, comprenant le chauffage dudit boîtier avant qu'il soit monté.

14. Procédé de compensation de la température (300) dans lequel un élément de compensation de la température est utilisé comme élément de compensation de la dilatation thermique comprenant :
la dilatation d'un élément plat (310) radialement lors de changements de température,
le déplacement d'un dispositif de liaison incliné radialement (320),
dans lequel la dilatation radiale dudit élément plat est convertie en un mouvement orthogonal (330) par rapport audit élément plat soulevant ou abaissant un boîtier enfermant ledit élément plat en fonction de la température dudit élément de compensation de la température.

15. Utilisation d'un élément de compensation de la température selon l'une quelconque des revendications 1 à 10 ou du procédé de compensation de la température selon la revendication 14, pour compenser un assemblage sensible à la température.
